# EUROPEAN PATENT APPLICATION

(11) **EP 3 254 649 A1**
(43) Date of publication of application: **13.12.2017**
(21) Application number: 15712390.2
(22) Date of filing: 03.02.2015
(51) Int. Cl.: A61F 6/00, A61F 6/04, A61F 6/06

(54) **PACKAGING FOR CONDOM**

(71) Applicant: Peña Arana, Javier, 48940 LEIOA (Bizkaia) (ES); Nijholt, Hendrik Johannes Karl, Medellin (CO)
(72) Inventor: PEÑA ARANA, Javier, E-48940 LEIOA (Bizkaia) (ES)
(74) Representative: Cueto, Sénida
(86) International application number: PCT/ES2015/070072
(87) International publication number: WO 2016/124798

(57) **Abstract**

Package (1) for a condom provided with a main compartment (6) for housing a condom (2), and an additional compartment (7) separate from the main compartment (6) and having a disposal element (10) that enables the condom (2) to be disposed of after use in an environmentally friendly manner. The invention provides a package (1) with integrated components, easy for the user to handle and without excessively increasing manufacturing costs compared to a standard commercial individual package. The invention also relates to a method of disposing of the condom (2) after use by the correct use of the package (1) described.

## Description

### Technical field

The invention relates to a package of the kind of unitary packages that house a condom or contraceptive sheath, and also to a method for disposing a condom after use.

### Prior art

Masculine or feminine condoms or contraceptive sheaths, which are a widely used barrier contraceptive method to prevent unwanted pregnancies and prevent the spread of sexually transmitted diseases, are known in prior art. Condoms are considered to have a proved efficiency, provided that the condom complies with appropriate quality specifications and is used correctly. Normally, a condom contains additional active substances, such as a certain amount of lubricant and/or spermicide.

Currently, condoms are a medical product and as such are subject to the corresponding health regulations of each country or region. These health regulations regulate the marketing conditions, quality controls and other requirements that condoms must comply with for sanitary approval, in order to ensure suitable reliability in use. For example, at the time of filing of the present patent application, the regulation governing technical and sanitary conditions that condoms must meet in order to be marketed in Spain, whether or not they contain spermicides or other active substances, is Royal Decree 414/1996 of 24/04/1996 which, in turn, is a transposition of European directive 93/42/EEC. By virtue of this current regulation, since June 1998 no health products are allowed to be sold in the European Union without a corresponding CE marking in accordance with the legislation in force. More particularly, in regards to packaging and labelling instructions, the regulation states that the condom is a single use device and that it must be disposed of in an individual container or package. The individual package must be hermetic or leak-proof, to ensure the integrity and preservation of the condom and the possible additional active substances, and the manufacturer identification, production batch number and condom expiration date must be shown on the individual package. One or more individual packages are usually arranged in a commercial container, e.g. a box, which must provide the same information as the individual package and, in addition, the number and type of condoms contained in the commercial container, an indication of potential additional active substance contents, references to the regulatory approval, and directions or instructions for correct conservation, use, and disposal or destruction of the condom after use. These instructions may appear on the commercial container itself or on a separate sheet provided inside the container. Thus, health authorities can refuse approval of a condom when its packaging is unsuitable for maintaining the technical and sanitary characteristics of the condom until its time of use or when labelling is insufficient to ensure correct use of the condom.

In practice, despite the current scenario described in the paragraphs above, disposal of the condom after use still presents several difficulties. The desirability of removing the condom as soon as possible after ejaculation is known, without this necessarily implying termination of the sexual encounter. Therefore, and at that special moment, the problem arises of what to do to quickly dispose of the used condom, with its unsightly appearance and impregnated with body fluids (semen, vaginal secretions, lubricants, etc.), so that the sexual encounter is minimally interrupted and there is a minimal loss of spontaneity.

A frequent domestic solution consists in placing the used condom on a home-made provisional wrapper or cover, such as a paper tissue or strip of toilet paper of the kind that is normally available in households. This provisional wrapper or cover, together with the condom, must later be thrown into a domestic waste bin. However, there are several drawbacks to this solution. On the one hand, the use of this provisional wrapper or home-made paper might be disagreeable and distasteful for the user because it interferes with the sexual encounter. On the other hand, the solution is not too hygienic since fluids from the used condom might leak onto or damage the surfaces on which the provisional wrapper or paper with the used condom tends to be placed temporarily. In addition to these drawbacks, disposing of the paper with the used condom by depositing it in the domestic waste bin might cause certain users a sense of shame or lack of privacy in the presence of third parties, who might see the discarded condom in the rubbish. In view of these drawbacks, the chances are high that the used condom will end up being thrown into a toilet instead of into the household waste bin. Throwing the used condom down the toilet is not an acceptable method of disposal, since discarded condoms can cause problems in the sanitary sewers and, even more seriously, cause environmental pollution problems. The drawbacks associated with disposing of the used condom are frequently exacerbated when sexual intercourse takes place outside the user's habitual residence, in hotels, vehicles, other people's houses or in public spaces such as parks or beaches. In these cases, it is even more likely that the used condom ends up in a toilet, on the street or any other inappropriate place, far from a rubbish container that would allow the condom to be otherwise collected by the municipal service and thrown into a controlled landfill site.

Alternatively to the provisional wrapper home-made paper solution or the like, several devices, cleaning kits or hygiene packs, greater or small in size and complexity, are known, having optional features to help remove a condom after use, clean the fluids generated during sexual intercourse and/or hold the used condom. Some of these devices or kits can optionally be commercially supplied together with one or more individual packages of condoms. Nonetheless, these devices are not always effective at avoiding the above problems, since they usually rely on the responsibility of the user, or have shapes that might lead to doubts or security issues when using the condom. Moreover, these devices lead to a greater or lesser increase in the purchase cost of the condom. Cited by way of example is patent application US5638949, disclosing a device to dispose of a used condom, which includes a container that can be closed to form a receptacle for the used condom.

An objective of the invention is to design a package or container for a condom that provides a practical alternative in order to facilitate disposal of the condom after use. In addition, the invention should provide a package which is easy and convenient for the user to handle, without excessively increasing the package production costs.

### Brief description of the invention

An object of the present invention is a package intended to hold at least one condom. Like other known unit or individual packages approved for sale, the package according to the invention is provided with two main sides opposite each other and delimited by an edge that is common to both, where the main sides and the edge delimit a sealed main compartment. The package according to the invention is unique in that it comprises at least one additional compartment separate from the main compartment and delimited by at least one of the two main sides and an additional side, where the additional side is arranged parallel to both main sides, so that the additional compartment is arranged parallel to the main compartment and with the additional compartment at least partially superimposed over the main compartment transversally to the package. The additional compartment of the package according to the invention has the additional particularity that it comprises an access area to allow access from outside to a disposal element arranged inside the additional compartment and shaped to protrude outward from the additional compartment.

The condom is initially stored or housed inside the sealed main compartment to suitably preserve its integrity. The feature of having a second additional compartment, integrated or incorporated into the condom package itself and provided with a disposal element that can be extracted from the additional compartment, enables the condom to be inserted into the disposal element after use. Therefore, the invention provides an individual package for a condom that, while maintaining a very similar appearance to known individual packages on the market and without the need for additional elements outside the individual package, has been modified, adapted or shaped to implement the additional functionality of enabling the condom to be stored after use and facilitating its disposal or destruction.

The package according to the invention has the important advantage of providing immediate availability of a disposal medium or element to place the condom in after use and later dispose of safely. As the disposal element is incorporated or integrated into the individual condom package itself, the drawback that the user must necessarily remember to have or prepare, prior to the sexual encounter, a paper tissue, strip of paper or other provisional home-made container, or some other type of specific external device for disposal of the condom, in which to deposit the condom after ejaculation, disappears. The user has easy, convenient access to the disposal element by simply accessing it through the access area and extracting it from the additional compartment. In addition, the disposal element of the package according to the invention enables the unsightly appearance of the condom after use to be concealed and, in a simple way, prevents condom fluids from making unwanted contact with items around the user. Thus, the user will feel less concerned about disposing of the used condom immediately, will not feel the need to dispose of the condom hastily down the toilet and its final disposal will be in a slow, relaxed manner.

The separate arrangement of the main and additional compartments in the package of the invention enables the necessary initial isolation of the condom to be maintained when it is housed in the main compartment. In addition, the parallel, superimposed position of the additional compartment in relation to the main compartment enables the additional functionality of disposing of the used condom to be provided by minimally modifying or altering the appearance and usual dimensions of an individual condom package of the type well-known on the market. These features mean that the invention is easily adaptable to a majority of individual packages sold on the market, with simple changes in the manufacturing process of the individual packages and small cost increases. Likewise, the individual or primary packaging of the condom is altered minimally, which facilitates compliance with regulations for sanitary approval. These advantages are all of major importance from a commercial point of view.

In a preferred embodiment, the disposal element has sufficient capacity to simultaneously hold the used condom and all other components of the package, enabling joint elimination of both condom and package. In short, the invention provides an individual package for a condom whose components are integrated into one unit and which forms a practical solution for disposing of the condom after use, with this solution also being environmentally friendly.

### Brief description of the drawings

Details of the invention can be seen in the accompanying drawings, which do not seek to restrict the scope of the invention:
- Figure 1 shows a front view of an embodiment of the package according to the invention.
- Figure 2 shows a front view of the package of Figure 1, illustrating the removal of the condom housed in the main compartment towards the outside of the package.
- Figure 3 shows a front view of the lower half of the package of Figure 1, in which the internal shape of the package can be seen in greater detail.
- Figure 4 shows a front view of the package of Figure 1, illustrating the removal of the disposal element towards the outside of the additional compartment so that the used condom can later be inserted therein.
- Figure 5 shows a front view of the disposal element after the used condom and the other components of the package have been inserted therein.

### Detailed description of the invention

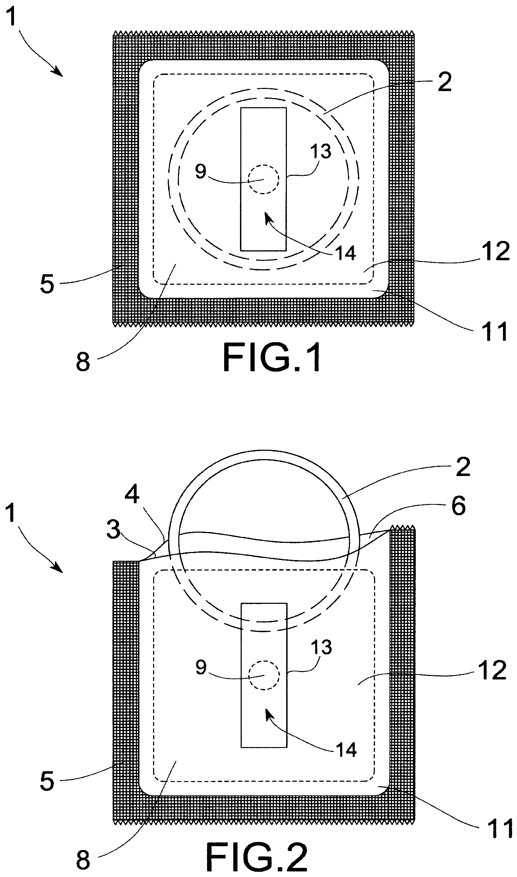

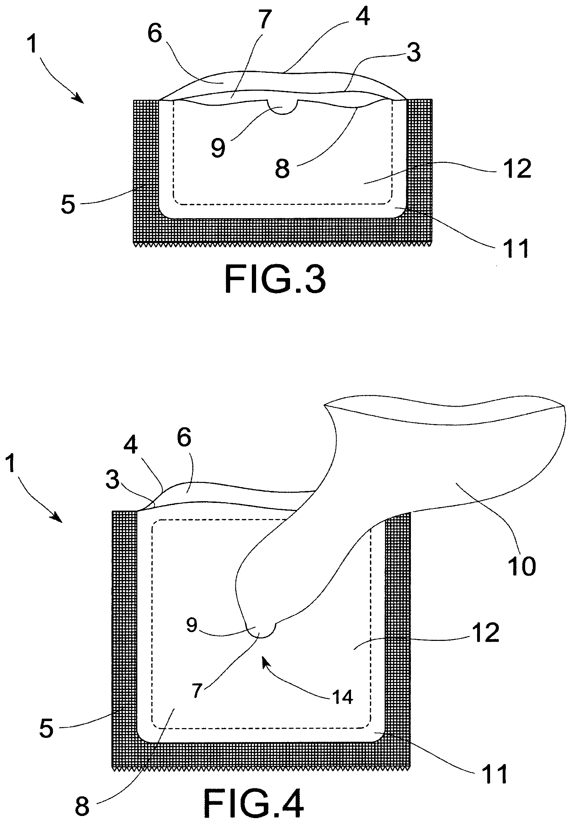

The invention relates to a package for a condom, of the type that is an individual, unitary or primary package known and used to hold a condom or contraceptive sheath. The invention also relates to a method of disposing of a used condom that uses the said package. Figure 1 shows a front view of an embodiment of the package according to the invention. In the said Figure 1, a condom is schematically depicted housed inside the package, while removal or extraction of the condom from the package is illustrated in Figure 2. The illustration of Figure 3 (in which the condom is not depicted) shows a front view of the lower half of the package of Figure 1, in which the internal shape of the package can be seen in greater detail. As shown in the figures, the package (1) has two main sides (3, 4) opposite each other and delimited by an edge (5) that is common to both main sides (3, 4). These main sides (3, 4) and the edge (5) delimit a hermetically sealed or leak-proof main compartment (6) for initially holding a condom (2). As also shown in Figure 3, the package (1) includes an additional compartment (7) isolated or separated from the main compartment (6) and delimited by the main side (3) and by an additional side (8). The additional side (8) is arranged parallel to both main sides (3, 4) of the main compartment (6). In this way, both compartments (6, 7) are also arranged parallel, with the additional compartment (7) superimposed over the main compartment (6) transversally to the package (1), as shown in Figure 3. Moreover, the additional compartment (7) includes an opening element or an access area (9) to allow the user to access, from outside, a disposal element (10) arranged inside the additional compartment (7). This disposal element (10) (not represented in Figures 1, 2 and 3) is shaped to protrude outward from the additional compartment (7). In the embodiment shown in the figures, the main side (3) has the added functionality of acting as a partition or separator element between the main and additional compartments (6, 7). Separation or isolation between both compartments (6, 7) is important for conservation, security and, ultimately, for correct use of both the condom (2) and the disposal element (10).

In addition, in the embodiment of Figures 1 to 3, the additional compartment (7) provides a housing whose capacity or size is slightly smaller than the capacity of the main compartment (6). As illustrated in Figure 2, in a similar way to other known condom packages, the user can proceed to open the main compartment (6) in order to extract the condom (2) by, for instance, tearing one side of the edge (5). In the package (1) according to the invention, the feature that the additional compartment (7) that holds the disposal element (10) is smaller in size than the main compartment (6) enables opening of the main compartment (6) in such a way that the additional compartment (7) remains closed and in such a way that the disposal element (10) remains protected inside it.

Figure 4 shows a front view of the package (1) of Figure 1, with the disposal element (10) shown outside the additional compartment (7) to enable subsequent insertion of the used condom (2) inside this disposal element (10). In the embodiment in the figures, the disposal element (10) is specifically an expandable bag which is initially folded inside the additional compartment (7). The extraction and subsequent opening of the disposal element (10) outside the additional compartment (7) enables the available space in the package (1) to be expanded or extended so that the condom (2) can be inserted after use.

Optionally, the additional side (8) extends to the edge (5) and the additional compartment (7) includes a connection area (11) located in an area very close or adjacent to the edge (5) and an inner space (12) to house the disposal element (10). The additional side (8) is connected to the main side (3) in this connection area (11) but the additional side (8) is separated or not connected to the main side (3) in the inner space (12). The connection or adhesion between the additional side (8) and the main side (3) around the connection area (11) can be performed by gluing or other means. With this configuration, the edge (5) of the package (1) is common to both compartments (6, 7) and is implemented by means of a sealed area or strip stretching around the perimeter edge of the package (1). In this way, the main sides (3, 4), the additional side (8) and the edge (5) form a single part, thereby simplifying production of the package (1). In addition, the inner space (12) that houses the disposal element (10) is conveniently smaller in size than the main compartment (6), thereby enabling, as shown in Figure 2, opening of the main compartment (6) in such a way that the additional compartment (7) remains closed and the disposal element (10) remains protected inside. Later, the disposal element (10) can be extracted independently through the access area (9), as shown in Figure 4.

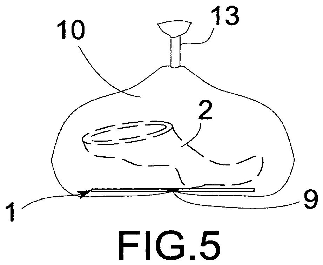

Preferably, in order to allow disposal of the entire assembly made up of the package (1) and the condom (2), the disposal element (10) has sufficient capacity to hold, when this disposal element (10) is extended, not only the condom (2) after use but also the other elements that make up the package (1), i.e. the main sides (3).4), the additional side (8) and the edge (5). Figure 5 illustrates the insertion inside the disposal element (10) of both the used condom (2) and the other components of the package (1).

Optionally, the disposal element (10) is connected, glued or attached to at least one of the sides that delimit the additional compartment (7). In the embodiment in the figures, the disposal element (10) is glued to the main side (3). This optional feature has the advantage of preventing separation of the disposal element (10) from the rest of the package (1), after its extraction from the additional compartment (7), preventing it from falling or being lost. This advantage is of great importance from an ecological or environmental point of view because it minimizes the risk of the components of the package (1) ending up being disposed of by the user independently of the disposal of the condom (2). As the disposal element (10) remains glued to one of the sides of the package (1), it makes it easy for the user to simply and naturally insert not only the used condom (2) but also the other components of the package (1) inside the disposal element (10). After extraction of the disposal element (10), the user can turn this disposal element (10) over so that the main sides (3, 4), the additional side (8) and the edge (5) are confined inside the disposal element (10) when it is turned over. After this turning operation, the condom (2) is also inserted inside the disposal element (10). Other embodiments of the invention are contemplated in which the disposal element (10) is separable or independent of the package (1) once that it has been extracted from it.

Additionally, the dimensions of the package (1) according to the invention are preferably small and similar to those of a standard individual package suitable for a commercial condom, so the assembly formed by the condom (2) and the package (1) is sanitarily approvable. In addition to the commercial advantages already mentioned in the brief description, maintaining a very similar dimensional configuration and external appearance to known individual commercial packages provides some additional advantages; unlike other solutions in the state of the art, consisting of more complex independent boxes or containers with motley appearances, dimensions and formats, the package (1) according to the invention facilitates identification of the product by the user and avoids doubts about its use and/or security concerns when using the condom (2).

Optionally, the disposal element (10) is perfumed. Being endowed with this optional feature, the package (1) according to the invention, as well as serving to conceal the unsightly appearance of the condom (2) after use, enables pleasant scents to be provided that help to neutralize the smell of body fluids and of the condom (2) material itself.

The invention also contemplates optional embodiments in which the disposal element (10) is recyclable or biodegradable.

Also optionally, the disposal element (10) is a compostable bag.

Due to the fact that the disposal element (10) provides the package (1) according to the invention with a larger available surface area for implementing advertising messages and/or graphic decorative or informative items, the disposal element (10) can optionally include graphical and/or advertising items. This optional feature gives the package (1) significant commercial possibilities.

Optionally, the package (1) includes a sealing element (13) to close off the access area (9), thereby preventing the withdrawal of the disposal element (10) to the outside of the additional compartment (7). In the embodiment of the package (1) shown in the figures, the sealing element (13) is a simple, adhesive-type element (such as a sticker) and the access area (9) is, more specifically, a hole made or cut in a central area (14) of the additional side (8). The central position of the access area (9) enables balanced withdrawal of the disposal element (10), facilitates the manoeuvre of turning over the disposal element (10) and minimizes the risk of deterioration of the disposal element (10), for example, with the edges of the edge (5), which frequently tend to have sharp edges. Moreover, the presence of the sealing element (13) provides a simple and readily available means of closing the disposal element (10) after the condom (2) and the other components of the package (1) have been inserted inside it. In this way, the risk of the disposal element (10) remaining open is minimised. Thus, the sealing element (13) also contributes towards providing an environmentally friendly package (1) so that all the elements that make up the package (1) according to the invention, including the sealing element (13), play a role, form a unit and are useful for this ecological purpose.

Embodiments of the invention, different from those represented in Figures 1 to 5, in which the access area (9) is not centrally positioned, are also contemplated. Embodiments in which the disposal element (10) has other shapes or is equipped with additional elements to facilitate closure (string, zip fastener, plasticised wire or other) are also contemplated, provided that the disposal element (10) is suitable for insertion into the additional compartment (7) of the package (1) without it compromising the integrity or security of the condom or for the integrity or security of the disposal element (10) itself.

In a second embodiment not represented in the figures, the access area (9) of the package (1) is located or implemented in a peripheral area adjacent to the edge (5) and the sealing element (13) is the edge (5) itself. This second embodiment of the package (1) increases its simplicity and makes the additional compartment (7) more leak-proof, thereby contributing to preserving the aromas and other products that might be associated with the disposal element (10). In this case, both compartments, main (6) and additional (7), open simultaneously by means of the edge (5).

In summary, the invention provides an individual package (1) for a condom (2) and a disposal method that facilitate disposal of a condom (2) after use in a clean, discrete, secure and environmentally friendly way.

## Claims

1. Package (1) for holding at least one condom (2), provided with two main sides (3, 4) opposite each other and delimited by an edge (5) common to both main sides (3, 4), where said main sides (3, 4) and the edge (5) delimit a sealed main compartment (6), **characterised in that** it comprises
- at least one additional compartment (7) separate from the main compartment (6) and delimited by at least one of the two main sides (3, 4) and by an additional side (8), where said additional side (8) is arranged parallel to both main sides (3, 4) so that the additional compartment (7) is arranged parallel to the main compartment (6), with the additional compartment (7) at least partially superimposed over the main compartment (6) transversally to the package (1), and where
- the additional compartment (7) includes an access area (9) to enable access from outside to a disposal element (10) arranged inside the additional compartment (7) and configured to protrude outwardly from said additional compartment (7).

2. Package (1), according to claim 1, **characterized in that** the additional side (8) extends to the edge (5), and **in that** the additional compartment (7) comprises a connection area (11) located in an area adjacent to the edge (5), the additional side (8) being connected to one of the main sides (3, 4) in said connection area (11), and an inner space (12) for housing the disposal element (10), with the additional side (8) not being connected to the main sides (3, 4) in said inner space (12).

3. Package (1), according to claim 1, **characterized in that** the main sides (3, 4), the additional side (8) and the edge (5) form a single part.

4. Package (1), according to claim 1, **characterized in that** the disposal element (10) has sufficient capacity to simultaneously hold the condom (2) after use, the main sides (3, 4), the additional side (8) and the edge (5).

5. Package (1), according to claim 1, **characterized in that** the disposal element (10) is connected to at least one of the sides that delimit the additional compartment (7).

6. Package (1), according to claim 1, **characterized in that** the dimensions are small and approximately similar to those of an individual standard package suitable for housing a commercial condom (2), with the assembly formed by the package (1) and the condom (2) being sanitarily approvable.

7. Package (1), according to claim 1, **characterized in that** the disposal element (10) is a bag and said bag is folded inside the additional compartment (7).

8. Package (1), according to claim 1, **characterized in that** the disposal element (10) is perfumed.

9. Package (1), according to claim 1, **characterized in that** the disposal element (10) is recyclable or biodegradable.

10. Package (1), according to claim 1, **characterized in that** the disposal element (10) is compostable.

11. Package (1), according to claim 1, **characterized in that** the disposal element (10) includes graphical and/or advertising items.

12. Package (1), according to claim 1, **characterized in that** the access area (9) includes a hole made in a central area (14) of the additional side (8).

13. Package (1), according to claim 1, **characterized in that** a sealing element (13) is included to close off the access area (9), thereby preventing the withdrawal of the disposal element (10) to the outside of the additional compartment (7).

14. Package (1), according to claim 13, **characterized in that** the sealing element (13) is of adhesive type.

15. Package (1), according to claim 13, **characterized in that** the access area (9) is implemented in a peripheral area adjacent to the edge (5) and the sealing element (13) is the edge (5) itself.

16. Method for disposal of a used condom, **characterised in that** it comprises the steps of:
- having an assembly that comprises a package (1) according to claim 1 and a condom (2) housed in the main compartment (6) of the package (1),
- opening the main compartment (6) of the package (1) to extract the condom (2) for use,
- opening the additional compartment (7) of the package (1) to access the disposal element (10),
- extracting the disposal element (10) towards the outside of the additional compartment (7),
- opening the disposal element (10),
- Inserting the condom (2), after use, inside the disposal element (10),
- closing the disposal element (10),
- disposing of the disposal element (10).

17. Method, according to claim 16, **characterized in that** the disposal element (10) of the package (1) has sufficient capacity to simultaneously hold both the used condom (2) and the main sides (3, 4), the additional side (8) and the edge (5) of the package (1), and wherein it includes the additional step of inserting the main sides (3, 4), the additional side (8) and the edge (5) inside the disposal element (10) after the step of opening the disposal element (10).

18. Method, according to claim 17, **characterized in that** the insertion of the main sides (3, 4), the additional side (8) and the edge (5) inside the disposal element (10) is performed by turning over the disposal element (10) on itself.

19. Method, according to claim 16, **characterized in that** the package (1) includes a sealing element (13) to close off the access area (9), thereby preventing the withdrawal of the disposal element (10) to the outside of the additional compartment (7), wherein the step of opening the additional compartment (7) is performed by withdrawing said sealing element (13) and wherein said sealing element (13) is also used for the step of sealing the disposal element (10).

20. Method, according to claim 16, **characterized in that** the step of disposing of the disposal element (10) is performed in an environmentally friendly manner.
